(19) Europäisches Patentamt

European Patent Office

Office européen des brevets

(11) **EP 1 100 867 B1**

(12) **EUROPEAN PATENT SPECIFICATION**

(45) Date of publication and mention
of the grant of the patent:
**01.12.2004 Bulletin 2004/49**

(51) Int Cl.$^7$: **C12M 1/107**

(86) International application number:
**PCT/IE1999/000076**

(21) Application number: **99933098.8**

(22) Date of filing: **26.07.1999**

(87) International publication number:
**WO 2000/006693 (10.02.2000 Gazette 2000/06)**

(54) **Digester**

FAULANLAGE

DIGESTEUR

(84) Designated Contracting States:
**AT BE CH CY DE DK ES FI FR GB GR IE IT LI LU
MC NL PT SE**

(30) Priority: **28.07.1998 IE 980629**

(43) Date of publication of application:
**23.05.2001 Bulletin 2001/21**

(73) Proprietors:
• **Heslop, Victoria Ann
Ballymacarbry, County Waterford (IE)**
• **Murcott, Andrew James
Shropshire S79 5HP (GB)**

(72) Inventors:
• **Heslop, Victoria Ann
Ballymacarbry, County Waterford (IE)**
• **Murcott, Andrew James
Shropshire S79 5HP (GB)**

(74) Representative:
**Gordon, Naoise Padhraic Edward et al
Murgitroyd & Company
165-169 Scotland Street
Glasgow G5 8PL (GB)**

(56) References cited:
FR-A- 2 305 113        FR-A- 2 381 980
FR-A- 2 388 487        FR-A- 2 474 474
FR-A- 2 513 481        US-A- 4 439 315
US-A- 5 500 306        US-A- 5 583 045

• DATABASE WPI Section Ch, Week 9148 Derwent
Publications Ltd., London, GB; Class C03, AN
91-351089 XP002120994 & JP 03 236724 A
(SUMITOMO SEIKA CHEM CO LTD), 22 October
1991 (1991-10-22)
• DATABASE WPI Section Ch, Week 9804 Derwent
Publications Ltd., London, GB; Class D15, AN
98-036002 XP002120995 & JP 09 289836 A
(TSURUSHI T), 11 November 1997 (1997-11-11)
• PATENT ABSTRACTS OF JAPAN vol. 008, no.
140 (M-305), 29 June 1984 (1984-06-29) & JP 59
038554 A (SEKISUI KOUJI KK), 2 March 1984
(1984-03-02)

# EP 1 100 867 B1

**Description**

This invention relates to a digester and more particularly to an integral anaerobic digester/greenhouse and to a method of anaerobic digestion.

Anaerobic digestion is widely used to treat liquid and solid organic wastes. For example, anaerobic digestion is widely used in the treatment of sewage, farm animal waste such as slurry or chicken litter, household rubbish or food processing waste. In anaerobic digestion, energy is released in the form of methane gas. The methane gas can be burnt to produce heat or it can be used to generate electricity for example in a combined heat and power generator. In this manner the methane gas can therefore generate electricity and heat to supply the energy demands of the digester while excess energy can be used as a source of energy for ancillary buildings and the like.

In known anaerobic digestion processes, the waste to be treated is usually in the form of a thin aqueous liquid, a thicker liquid or a solid. Thicker liquids can be allowed to settle to separate into a thicker portion and a supernatant. The thicker portion containing a high dry matter content is loaded into the digester. If desired, thinner or supernatant liquids can also be loaded into the digester as required or can be subjected to a thickening process or settled. The thickening process results in a raw feed sludge having a higher solids content than the thinner or supernatant liquid and an aqueous byproduct. The raw feed sludge is loaded into the anaerobic digester and the nutrient rich water byproduct usually requires further treatment before being discharged or may be spread on land. In addition, following digestion in the digester and separation a fibre and a nutrient rich aqueous byproduct is also formed which also requires disposal. The fibre can be composted to form a soil conditioner.

The water byproducts can undergo further treatment e.g. in aeration plants in cold greenhouses where pond weed is grown on the water byproduct to remove the nutrients from the water byproduct. Moreover, the running costs incurred in order to treat dirty water byproducts on-site are expensive and accordingly such byproducts are usually land spread. However, transportation costs, generally referred to as "tankerage", can be significant or indeed exorbitant where large quantities of waste materials are involved.

Known anaerobic digestion methods also suffer from the disadvantage that heat and gases such as waste ethane can be discharged in large quantities into the atmosphere.

In general, optimum use of by-products cannot be achieved with know anaerobic digestion methods at or near the proposed location of a digester thereby compromising the economic viability of the digester. Accordingly, under exploitation of digesters can result.

FR-A-2 305 113 and US-A-5,500,306 disclose systems for producing methane from organic waste which comprise an anaerobic digester located within a greenhouse. In these systems, however, the configuration of the digester tank within the greenhouse does not allow a direct heat transfer between tank and greenhouse.

FR-A-2 388 487 discloses an agricultural station which comprises a greenhouse having at least a portion of its walls and roof transparent, and a device for melting snow deposited on the roof and heating the greenhouse which works with solar energy and/or energy produced by combustion of biogas. FR-A-2 474 474 discloses a system for improving urban sanitation by producing energy from organic waste. In these systems, the anaerobic digester is separated from the greenhouse.

An object of the invention is to overcome the problems of the prior art.

A further object of the invention is to provide an integral digester/greenhouse.

A further object of the invention is to provide a process for anaerobic digestion which overcomes the problems of the prior art.

According to the invention there is provided an integral digester and greenhouse comprising a greenhouse and a digester, wherein the digester is an anaerobic digester and is located within the greenhouse to allow direct heat exchange between the digester and the greenhouse.

The digester comprises a digester tank for organic waste materials, which is uninsulated or partially insulated.

Suitably the greenhouse surrounds the digester. Advantageously, the greenhouse comprises a circular greenhouse and the digester is centrally located in the greenhouse. Alternatively, the digester is located in the greenhouse to define a higher temperature portion and a lower temperature portion in the greenhouse.

Suitably, the greenhouse comprises a side wall defined by uprights and a roof defined by beams, the beams extending between the uprights and the digester. Advantageously, the beams are supported on a gantry defined on the digester.

Suitably, the greenhouse comprises a glazing skin. Preferably, the skin comprises glass or plastics. Suitably the plastics is selected from the group comprising polyethylene, glass reinforced polyester and polyvinyl chloride.

In a preferred embodiment of the invention, the greenhouse comprises an internal lagoon for receiving liquid. Suitably, the lagoon comprises an inlet for receiving liquid and an outlet for discharging liquid from the lagoon.

Preferably, the greenhouse comprises a service area from which the integral digester and greenhouse may be serviced. Preferably, a combined heat and power unit is located in or adjacent the service area. Suitably, the service area further houses a separator for the digester. Advantageously, control means for controlling the combined heat and power unit, the separator and the digester is housed in the service area.

The invention also extends to a method for digesting organic material and heating a greenhouse, the method comprising digesting the organic material in a digester and locating the digester to be in heat exchange co-operation with the greenhouse, wherein the method comprises locating an anaerobic digester within the greenhouse for facilitating direct heat exchange between the digester and the greenhouse.

Suitably, at least a portion of the energy is converted to electrical energy. Advantageously, the energy comprises heat.

Suitably, the organic material is separated prior to or following digestion of the organic material.

Advantageously, an aqueous supernatant is derived from the organic material prior to digestion of the organic material by separating the organic waste. Suitably, a fibre and a nutrient rich liquid are derived from the organic material following digestion of the organic material. Preferably, the organic waste is separated by thickening, settling, pressing or centrifuging.

Advantageously, the liquor or liquid is placed in a lagoon in the greenhouse for purification. Preferably, the supernatant is purified by cultivating an organism on the supernatant in or on the lagoon. Advantageously, the organism comprises vegetation. Suitably, the vegetation comprises aquatic plants.

Preferably, the aquatic plants comprise water hyacinths.

Suitably, the water hyacinths are digested in the digester.

The integral digester and greenhouse of the invention optimises energy efficiency and achieves savings in capital costs compared with digesters of the prior art. The integral greenhouse/digester of the invention also reduces the amount of heat and carbon dioxide that is discharged into the atmosphere compared with anaerobic digesters of the prior art as the apparatus and method of the present invention facilitates the use of byproducts of the digestion process. Moreover, the heat generated by an anaerobic digester in a greenhouse makes possible greenhouse production of plants, fish and the like in the greenhouse with increased energy efficiency and minimal use of fossil fuels.

The integral digester and greenhouse also provides a cost-effective method of treating thin nutrient rich liquors in preference to the land spreading methods of disposal of the prior art.

In the integral greenhouse/digester of the invention, the side walls of the digester are not insulated to optimise heat transfer across the side walls thereby affording savings in the construction costs of the digester. The heat lost from the digester is therefore allowed to radiate into the body of the greenhouse to encourage horticultural or aquacultural growth. Heat radiation from the digester may be assisted by convection fans.

Moreover, heat generated within the greenhouse from sunlight can be absorbed and stored by the uninsulated digester for subsequent radiation.

The digester within the greenhouse can also serve as a heat storage unit to store heat produced by a combined heat and power generator where the heat is not required for other applications. The stored heat may then be radiated as required when heat produced by the combined heat and powered generator is required elsewhere. Accordingly, if desired, the digester in the digester/greenhouse arrangement of the invention can obviate the need for separate heat stores usually required to store excess heat from a combined heat and power generator. Such heat stores are in general prohibitively expensive, resulting in their lack of use and the resultant dumping of heat.

The apparatus and method in accordance with the invention is environmentally friendly in that the amount of heat and carbon dioxide discharged into the atmosphere from anaerobic digesters and their associated combined heat and power generators is also reduced. For example, location of a digester in combination with a combined heat and power unit within a greenhouse structure facilitates use of heat generated by the anaerobic digester and/or the combined heat and power unit.

The combined digester/greenhouse arrangement of the present invention also facilitates employment of the heat generated from the combined heat and power generator to dehydrate the fibre remaining after digestion to produce a commercially acceptable and economical nutrient rich soil conditioner. Alternatively, or in addition, excess electricity from the combined heat and power unit can be employed to power a dehumidifier to dehydrate the fibre.

The combined digester/greenhouse of the invention also minimises or eliminates the quantity of fossil fuel usually employed to heat the greenhouse.

Solar heat from the sun within the greenhouse serves to minimise loss of heat from the digester. In addition, the digester absorbs a portion of the solar heat and acts as a heat store for the solar heat until such time as the sunlight diminishes.

In addition, the digester can also reflect and radiate light to effect heating of the greenhouse. The amount of heat absorbed or light radiated (by the surface of the digester) is dependant upon the colour of the digester tank wall which may be varied in accordance with the desired heat absorbtion and/or light radiation characteristics. In addition, the amount of heat absorbed or light radiated by the digester tank is also dependant upon the angle of the sun and the glazing material employed in the construction of the greenhouse.

Accordingly, the combined greenhouse/digester of the invention optimises organic waste recycling by fully utilising co-generated heat and maximising the return from all byproducts. Moreover, the combined greenhouse/digester of the invention due to the use of all byproducts facilitates a reduction in greenhouse gas emissions associated with agricultural practices.

The invention will now be described by way of example only having regard to the accompanying drawings in which:

Fig. 1 is a side elevation of an integral digester/greenhouse in accordance with a first embodiment of the invention;

Fig. 2 is a plan view from above of the integral digester/greenhouse of Fig. 1, and

Fig. 3 is a plan view from above of a site layout of an integral digester/greenhouse in accordance with a second embodiment of the invention.

As shown in the drawings, an integral digester/greenhouse in accordance with the invention is generally indicated by the reference numeral 1. The integral digester/greenhouse 1 is adapted to effect anaerobic digestion of raw feed organic material within a greenhouse environment such that the greenhouse is heated by the energy generated during anaerobic digestion and heat transfer from a digester 2 in the integral digester/greenhouse 1.

More particularly, the integral digester/greenhouse 1 is made up of a digester 2 centrally located within a circular greenhouse 3. The digester 2 is substantially of conventional construction and is made up of a substantially cylindrical tank 4 having a circular base (not shown) and a side wall 5 upstanding from the base. The side wall 5 terminates at a quasi dome-shaped roof 6.

The tank 4 is formed from conventional digester tank materials such as steel. However, the tank side wall 5 is not insulated to enhance heat transfer between the tank 4 and the greenhouse 3 through the side wall 5. The roof 6 of the digester 2 is insulated in conventional manner.

The base of the tank 4 is in turn mounted on a concrete base 7 to support the tank 4 of the digester 2. The concrete base 7 is substantially circular in shape and is centrally located within the circular greenhouse 3. The concrete of the concrete base 7 is corrosion resistant should the concrete base 7 come into contact with corrosive liquids such as the aqueous byproduct of digestion as shall be explained more fully below.

The roof 6 of the tank 4 is made up of roof segments 8 which extend outwards from the central point of the circular roof 6. Openable and closable openings 9, 10 are defined in two segments of the roof segments 8 of the roof 6. The openings 9, 10 facilitate access to the cylindrical tank 4 and release of gases and the like from the tank 4 as required.

A circumferential gantry 11 is mounted adjacent the roof 6 on the tank side wall 5. The gantry 11 facilitates access to the openings 9, 10 and to the roof 6 for maintenance purposes as required.

A ladder 12 extends downwards from the gantry 11 towards the base of the tank 4 adjacent the side wall 5 to facilitate access to the gantry 11.

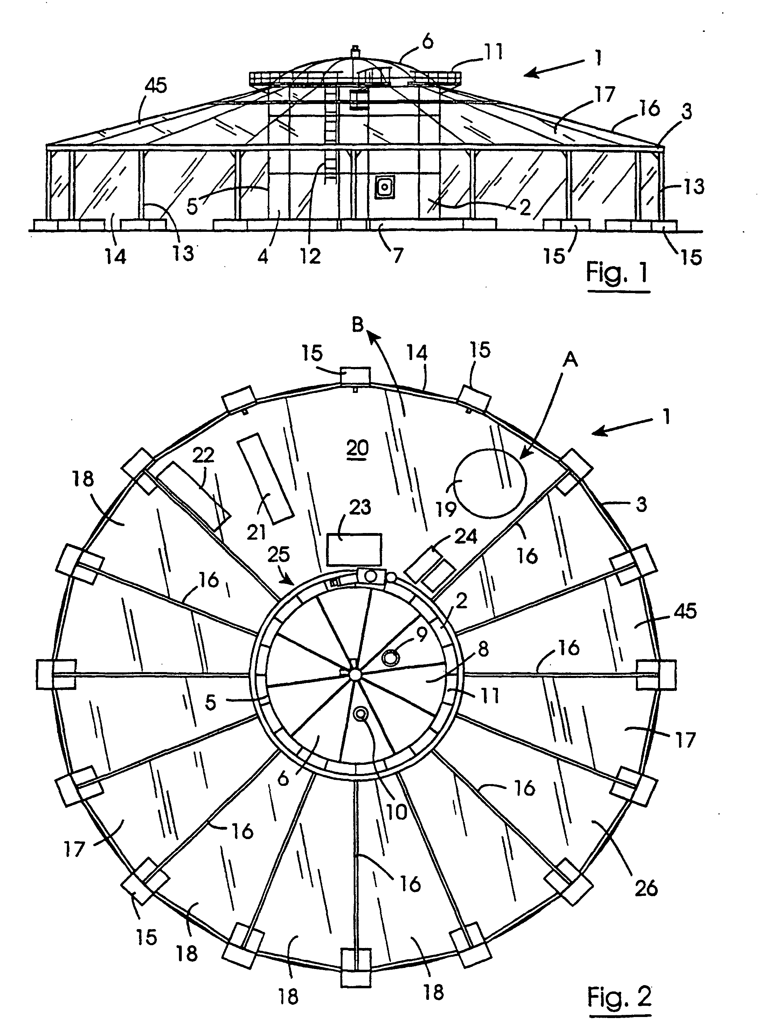

As indicated above, in the present embodiment, the digester 2 is centrally located within the circular greenhouse 3. The greenhouse 3 is circular in outline when viewed in plan from above as shown in Fig. 2 and is made up of circumferentially spaced apart greenhouse uprights 13 to define a greenhouse peripheral side wall 14 about the centrally located digester 2. The greenhouse uprights 13 are mounted in corrosion resistant concrete plinths 15 to support the greenhouse uprights 13. The concrete plinths 15 also serve to protect the greenhouse uprights 13 from corrosive materials such as the aqueous byproducts of digestion referred to above.

The greenhouse 3 is provided with a roof 17 defined by beams 16 which extend between the free ends of the greenhouse uprights 13 and the gantry 11. More particularly, the beams 16 are inclined downwards from the gantry 11 towards the free ends of the uprights 13 and are supported between the free ends of the uprights 13 and the gantry 11. Accordingly, greenhouse roof segments 18 are defined between the beams 16 of the roof 17.

In an alternative embodiment of the invention, the beams 16 are not mounted on the gantry 11 but can be supported adjacent the gantry 11 on uprights.

The greenhouse uprights 13 and the beams 16 are typically manufactured from aluminium or protected steel. A polythene skin 45 is mounted over the greenhouse uprights 13 and the beams 16 to fully define the greenhouse side wall 14 and the roof 17 to provide an enclosed greenhouse 3. In a preferred embodiment of the invention the polythene skin is formed from glass reinforced polyester (GRP). Alternatively, standard greenhouse glazing may be employed for the skin 45.

The roof 6 of the digester 2 stands proud of the greenhouse roof 17 and accordingly is not housed within the greenhouse 3. The roof 6 of the digester 2 is therefore insulated.

The digester 2 in combination with the integral greenhouse 3 defines an enclosed heatable substantially annular greenhouse area 26 about the central digester 2.

Generally, the integral digester/greenhouse 1 of the invention is oriented to maximise exposure to sunlight. However, the integral digester/greenhouse 1 of the invention will generally have a face which receives reduced exposure to sunlight e.g. a north face. For the purposes of the present embodiment a north or reduced sunlight face or portion of the integral digester/greenhouse 1 is generally indicated by the reference numeral 25. The north face portion 25 may be used as a service area to locate equipment and the like within the annular greenhouse area 26 while the remaining portion of the annular greenhouse area 26 is suitable for use in horticulture and/or aquaculture of plants, fish and the like where elevated temperatures are required for growth as shall be explained more fully below. Alternatively, the north face portion 25 can be used to house a shed or the like.

As shown in Fig. 2, the north face portion 25 of the annular greenhouse area 26 is provided with a pit 19 for receiving material to be digested by the digester 2 and a loading bay 24 disposed adjacent the tank 4 for loading material from the pit 19 into the tank 4. In an alternative embodiment of the invention the pit 19 can be replaced by a storage tank or the north face portion 25 can house both pits 19 and tanks 25.

In addition, a control unit 21 for controlling anaerobic digestion within the digester 4, a combined heat and power (CHP) unit 22 and any other associated equipment is located within the north face portion 25. The combined heat and power unit 22 serves to generate heat and electricity from methane gas produced during digestion in the digester 2.

Depending on the location of the digester 2 relative to the north face portion 25, the digester tank 4 may be insulated or partially insulated in the north face portion 25.

A separator 23 is also mounted adjacent the tank 4 of the digester 2 for separating digested material following digestion in the digester 2 into a dry matter or fibrous compost portion and a nutrient rich liquor.

Separation prior to digestion achieves the removal of water from the raw feed organic material while separation following digestion serves to remove fibre from a nutrient rich liquid.

The separator 23 can be of a standard type e.g. employing thickening, settling, pressing or centrifuging procedures.

A compost region, generally indicated by the reference numeral 20 for receiving the fibrous compost byproduct of the separator 23 is also located within the north face portion 25. Material from the compost region 20 may be removed from the north face portion 25 in the direction indicated by the arrow B as required. Similarly, organic material to be digested by the digester 2 may be received within the north face portion 25 in the direction indicated by the arrow A adjacent the pit 19 as required.

The greenhouse side wall 14 is also provided with the necessary entrances and exits (not shown) to facilitate receipt of organic waste and removal of compost as required. The entrances and exits also facilitate access to the control unit 21 and the combined heat and power unit 22 as required.

The annular greenhouse area 26 can be constructed as required in accordance with standard greenhouse constructions for horticultural and aquacultural purposes and the like. The annular greenhouse area 26 can be employed for horticultural and/or aquacultural purposes. Where the annular greenhouse area is employed for horticultural purposes, plants can be cultivated directly in the soil of the annular greenhouse area 26 or in containers. The annular greenhouse area 26 can also house a water filled lagoon in which aquatic plants can be cultivated. Alternatively, the lagoon could be employed to farm fish and the like. The lagoon is typically provided with inlets and outlets for receiving and discharging fluid to and from the lagoon. The water filled lagoon can be adapted to treat the nutrient rich supernatant or water byproducts resulting from digested and (separated) undigested organic wastes such as sewage, animal waste and the like. The corrosion resistant concrete plinths 15 and base 7 of the digester 2 and the uprights 13 respectively therefore prevent corrosion by the aqueous supernatants which can be corrosive in nature.

In use, where a lagoon for treating an aqueous byproduct is employed, sewage or the like requiring treatment may be thickened in a conventional manner. The sewage requiring digestion can be placed in the pit 19 for storage and subsequently loaded at the loading bay 24 into the tank 4 of the digester 2. The nutrient rich thin byproduct of the thickening process and/or settled liquid byproduct of the anaerobic digestion within the tank 4 is placed in the lagoon in the annular greenhouse area 26. Digestion of the waste material within the digester 2 is carried out in the usual manner with the exception that, as indicated above, the tank side wall 5 is uninsulated to maximise heat transfer from the digester 2 to the lagoon within the annular greenhouse area 26. Aquatic plants such as water hyacinths are typically placed in the lagoon to grow on the waste water byproduct to remove the nutrients from the waste water byproduct thereby purifying the waste water byproduct to facilitate discharge of the waste water byproduct in a safe and environmental fashion without giving rise to significant tankerage costs.

Heat transfer from the digester 2 and heat generated using the CHP generator 22 effects heating of the lagoon to facilitate growth of the water hyacinths and therefore water purification. The heat or energy generated can also be exploited to heat other structures as required. Similarly, the CHP generator 22 can be used to generate electricity for providing power to the digester 2. In addition, sunlight augments heating within the greenhouse 3 to accelerate growth of the water hyacinths and also to heat the digester 2.

Following growth of the water hyacinths within the lagoon, the water hyacinths may be harvested and in turn loaded into the digester 2 for digestion thereby giving rise to a continuous purification process. The product of anaerobic digestion within the digester 2 may then be spread on land etc. in the usual manner in a safe and environmentally friendly fashion.

As indicated above, the digester 2 of the invention therefore functions as an energy source and a heat sink to absorb excessive heat generated within the greenhouse 3 during exposure to sunlight and the like.

As indicated above, the lagoon within the annular greenhouse area 26 may simply be replaced by a standard greenhouse construction where the digester 2 of the invention is exploited for providing heat to and functioning as a heat store for horticultural purposes.

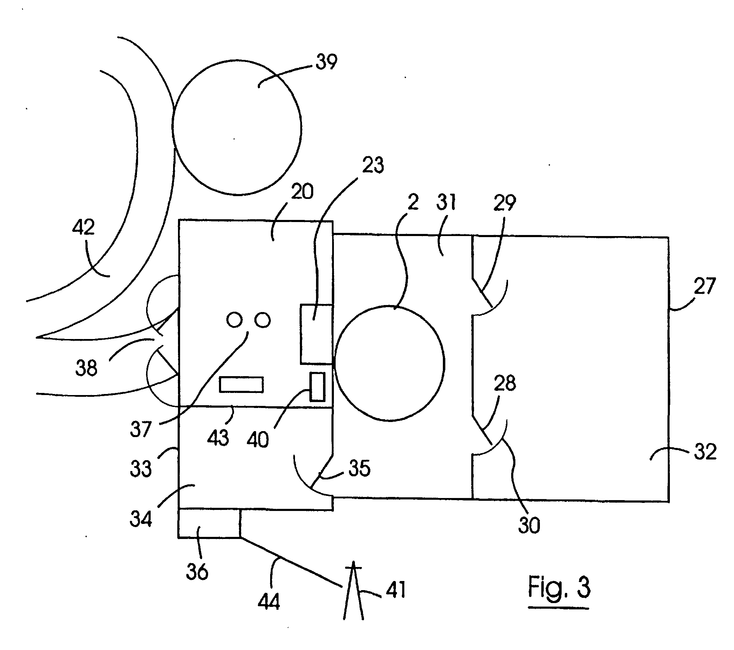

Fig. 3 shows a top plan view of a second embodiment of an integral digester/greenhouse 1 of the invention. The integral digester/greenhouse 1 of Fig. 3 is broadly similar to the integral digester/greenhouse 1 of Figs. 1 and 2 and accordingly, like numerals indicate like parts. Fig. 3 also shows a site layout for the integral digester/greenhouse 1.

As shown in the drawing, the integral digester/greenhouse 1 is made up of a substantially rectangular shaped greenhouse 27 portion and a shed portion 33 defined at the northern face 25 of the rectangular greenhouse 27. The rectangular greenhouse 27 is provided with a dividing wall 28 to divide the rectangular greenhouse 27 into a higher temperature portion 31 and a lower temperature portion 32. The higher temperature portion 31 houses the digester 2 and is communicable with the lower temperature portion 32 via a first communicating door 29 and a second communicating door 30 defined in the dividing wall 28. The communicating doors 29,30 also serve to facilitate heat transfer or heat equilibration between the higher temperature portion 31 and the lower temperature portion 32 as required.

The shed portion 33 is also divided by a shed dividing wall 43 to define a greenhouse store portion 34 for receiving products from the greenhouse 1 and a compost region 20 also adapted to accommodate various apparatus. The greenhouse store portion 34 is communicable with the higher temperature portion 31 of the rectangular greenhouse 27 via a greenhouse store door 35.

A combined heat and power unit 36 is located externally of the greenhouse store portion 34 and is communicable with an electricity network generally indicated by the reference numeral 41 via an electric cable 44. Accordingly, surplus electricity generated by the combined heat and power unit 36 from the combustion of methane from the digester 2 or the like may be communicated to the electricity network 41 via the electric cable 44.

The compost region 20 is broadly similar to the compost region 20 of the integral greenhouse/digester 1 of Figs. 1 and 2 and is provided with a reception area 37 for receiving material to be digested through main doors 38 from a liquor holding tank 39. Material to be digested in the digester 2 may be conveyed to the reception area 38 on a roadway 42. The compost region 20 is further provided with a pasteuriser 40 and a separator 23.

The integral digester/greenhouse 1 of Fig. 3 is operated in a broadly similar fashion to the integral digester/greenhouse 1 of Figs. 1 and 2. However, in the present embodiment, the rectangular greenhouse 27 may be operated so that plants, fish or the like requiring a high temperature may be grown in the higher temperature portion 31 of the rectangular greenhouse 27 while plants, fish or the like requiring ambient temperature may be cultivated in the lower temperature portion 32.

The shed 33 may also be provided with underground waste storage tanks (not shown). Radiation of heat from the digester 2 into the rectangular greenhouse 27 may also be assisted by convection fans (not shown).

In a further embodiment of the invention, the integral digester/greenhouse can be provided with multiple digesters 2. In addition, the digester(s) 2 can be located at ground level within the integral digester/greenhouse 1 or, alternatively, submerged below soil level dependant upon the construction of greenhouse and height requirements/restrictions. A channel could be defined in this embodiment about the digester 2 to facilitate radiation of heat. Alternatively, the digester 2 can be submerged below soil level in contact with the soil to facilitate heating of the soil for cultivating plants.

The greenhouse of the integral digester/greenhouse 1 can be of any desired shape and the present invention should not be construed as being limited to integral digester/greenhouses 1 having a particular shape.

In a further embodiment of the invention, the integral digester/greenhouse 1 of the invention may be provided with pipes for communicating between the combined heat and power unit 22 and the greenhouse to augment heating within the greenhouse.

In addition, the digester/greenhouse 1 of the invention can be provided with a translucent curtain around the digester 2 to screen the greenhouse 3 from heat loss from the digester 2 if desired and increase the heat storage capability of the digester 2.

The heat flow characteristics of a typical integral digester/greenhouse 1 in accordance with the present invention in which the digester 2 has a capacity of $1,400m^3$ and a diameter of 14m are as follows.

Heat losses from an insulated digester: $\sim$10-15Kw.

Heat losses from an uninsulated digester to the atmosphere at 0°C: $\sim$200Kw.

Heat losses to a greenhouse at 18°C ambient: $\sim$100Kw.

If the digester and greenhouse are at a constant temperature then the heat flow to the greenhouse from the digester will also be constant, except when the sun shines on the digester through the greenhouse.

**Heat Balance**

The digester feed volume (loading rate) is assumed to be 90m$^3$ per day maximum and the raw feed temperature is assumed to be 5°C.
It should be noted that raw feed heating can be supplied by heat exchangers situated inside the digester vessel 2.
Accordingly, the heat required to heat raw feed to 40°C (operating temperature of the digester) is calculated to be about 155Kw.
The digester operating temperature could be varied from 34°C to 42°C for a mesophytic digestion.

**Heat store characteristics of a typical integral digester/greenhouse.**

It is assumed that the digester 2 can operate with a 1°C temperature variation with no loss of process performance.
The temperature within the greenhouse is maintained by standard greenhouse temperature control means.
To raise the digester temperature by 1°C absorbs 1,650Kw hours of heat.
The heat can be absorbed from a plurality of sources. For example, during daylight where the greenhouse may not require the full output of a CHP unit 22, the available heat from the CHP unit 22 can be stored by the digester 2.
If this heat is absorbed during a 12 hour "day period" the heat demand by the digester 2 is increased by 137Kw for this period.
Consequently the "night period" heat is increased by 137Kw from the digester 2 and 137Kw from the CHP unit 22 giving a variation in available heat for the greenhouse between day and night periods (even if the digester is not allowed to drop below its "optimum" operating temperature).
Accordingly, the digester 2 has at least the following heat exchange capability:

| | |
|---|---|
| Raw feed: | 155Kw |
| Heat exchange through digester | 100Kw |
| wall to greenhouse: Heat required to increase the digester temperature by 1°C: | 150Kw |
| Total | 405Kw |

A typical CHP unit 22 for use in the present embodiment of the invention would generate 250Kw electricity for export to the electricity network 41.
The CHP unit 22 would also generate 450Kw of heat.

**Heat Exchanger Design**

Digester 2 heating is also provided (in addition to heating resulting from sunlight) by annular heat exchangers situated on the base of the inside of the digester tank.
Typically for a digester having a capacity of 1,400m$^3$ twenty-four heat exchangers arranged in two circles of sixteen and eight heat exchangers should suffice.
Such an arrangement provides an almost even distribution of heat over the base area of the digester 2.
The sidewall 5 of the integral digester/greenhouse 1 of the invention does not require insulation. Moreover, due to the location of the digester 2 within an integral digester/greenhouse 1, the heat generated by the digester 2 does not require piping or transmission by heat exchangers to a remote location as the heat is exploited within the integral digester/greenhouse 1. Moreover in the event of power failure, e.g where pumps and the like within conventional digesters 2 fail, heat within the digester 2 is only slowly lost due to the presence of the greenhouse 3 surrounding the digester 2. In addition, heat from the digester 2 can serve to heat the greenhouse and prevent crop loss. Accordingly, it is not necessary to provide expensive back-up pumps and the like to anticipate power failures. However, as will be appreciated by those skilled in the art, back-up pumps may be incorporated into the integral anaerobic digester/greenhouse of the invention if required.
In addition, the digester 2 can help to support the greenhouse 3 structure, thereby reducing the cost of construction of the greenhouse 3.
In effect, the digester 2 of the integral digester/greenhouse of the invention functions also as a solar heat storage tank whereby sunlight impinging on the digester tank side wall 5 through the greenhouse 3 raises the temper-

# EP 1 100 867 B1

ature of the digester 2 thereby preventing loss of heat from the digester 2. In addition, the digester 2 also serves to store heat.

The heat is stored within the tank 4 as a comparatively small temperature rise and is then subsequently released in the absence of solar heat at night.

The insolation rate of the sun is about 1.1Kw/m$^2$. However, as indicated above, the actual rate of heat absorption is dependant upon the angle of the sun and the colour of the digester tank side wall 5. For example, a steel digester tank 2 having a black side wall 5 with the sun at a 45° angle absorbs (0.7 x 1.1) Kw heat/m$^2$ of wall surface. Accordingly, in the embodiment of the invention described above where the digester tank 2 has a capacity of 1,400m$^3$ and a side elevation of 14 x 9m (if the total surface is included), the total potential heat absorbed is 0.77 x 126m$^2$ = 97Kw.

An advantage to a digester tank 2 having a black side wall is that the black colour of the side wall 5 enhances heat absorption into the digester tank 2. However, as the temperature of the contents of the digester tank 2 is controlled, the digester tank 2 serves as a heat exchanger to absorb the heat from the black side wall 5 so that the black side wall 5 is maintained at a constant temperature. In contradistinction, a black wall within a greenhouse which is not part of a digester 2 would simply continue to increase in temperature in sunlight giving rise to excessive heat within the greenhouse and potential damage to any crop within the greenhouse.

The greenhouse 3 is typically provided with a foundation wall which serves as a support for the greenhouse and also a bunding wall to retain any spillages from the digester 2 or the lagoon (where present).

The integral digester/greenhouse of the invention in combination with the aforementioned lagoon obviates the requirement of tankerage for aqueous byproducts requiring purification as the aqueous material can be purified in situ in the integral digester/greenhouse of the invention. The integral digester/greenhouse is also suitable for agricultural or aquacultural growth within the integral digester/greenhouse 1 thereby maximising the heat and power generated by the integral digester/greenhouse 1.

The dimensions of the integral digester/greenhouse 1 of the invention can be varied as required. For example, it is envisaged that the integral digester/greenhouse 1 of the invention could have dimensions ranging between 0.5 acres and 0.5 hectares.

The integral digester/greenhouse therefore has multiple and many applications in the area of waste management such as in animal farm byproducts, human sewage, slurry treatment, food processing wastes and the like.

The integral digester/greenhouse can be a permanent or temporary structure whereby the greenhouse 3 may be formed from a collapsible greenhouse structure. Generally, the dimensions of the digester will vary according to the output required.

Similarly, the combined heat and power unit may be selected in accordance with the requirements of the integral digester/greenhouse 1 of the invention. An example of a suitable combined heat and power unit is the Jenbacher Bio-Gas Engine. Separators and pasteurizers suitable for use in the present invention are available from Methanogen Limited.

## Claims

1. An integral digester and greenhouse comprising a greenhouse (3) and a digester (2), **characterised in that** the digester (2) is an anaerobic digester (2) and is located within the greenhouse (3), the anaerobic digester (2) comprising an uninsulated or partially insulated digester tank (4) to allow direct heat exchange between the digester (2) and the greenhouse (3).

2. An integral digester and.greenhouse as claimed in Claim 1 **characterised in that** the digester tank (4) comprises a base, an uninsulated side wall (5) upstanding from the base and a roof (6).

3. An integral digester and greenhouse as claimed in Claim 1 or 2 **characterised in that** the greenhouse (3) surrounds the digester (2).

4. An integral digester and greenhouse as claimed in Claim 3 **characterised in that** the greenhouse (3) comprises a circular greenhouse (3), and the digester (2) is centrally located in the greenhouse (3).

5. An integral digester and greenhouse as claimed in any preceding Claim **characterised in that** the digester (2) is located in the greenhouse (3) to define a higher temperature portion (31) and a lower temperature portion (32) in the greenhouse (3).

6. An integral digester and greenhouse as claimed in any preceding claim **characterised in that** the greenhouse (3) comprises a glazing skin (45) of plastics, which is selected from the group comprising polyethylene, glass reinforced

8

polyester and polyvinyl chloride.

7. An integral digester and greenhouse as claimed in any preceding Claim **characterised in that** the greenhouse (3) comprises a service area (25) from which the integral digester and greenhouse (1) may be serviced, a combined heat and power unit (22) being located in or adjacent the service area (25).

8. An integral digester and greenhouse as claimed in Claim 7 **characterised in that** a separator (23) for the digester (2) is housed in the service area (25).

9. A method for digesting organic material and heating a greenhouse, the method comprising digesting the organic material in a digester (2) and locating the digester (2) to be in heat exchange co-operation with the greenhouse (3), **characterised in that** the method comprises locating an anaerobic digester (2) comprising an uninsulated or partially insulated digester tank (4) within the greenhouse (3) for facilitating direct heat exchange between the digester (2) and the greenhouse (3).

**Patentansprüche**

1. Ein integraler Faulbehälter und ein Treibhaus, das ein Treibhaus (3) und einen Faulbehälter (2) beinhaltet, **dadurch gekennzeichnet, dass** der Faulbehälter (2) ein anaerober Faulbehälter (2) ist und sich innerhalb des Treibhauses (3) befindet, wobei der anaerobe Faulbehälter (2) einen nicht isolierten oder teilweise isolierten Faulbehältertank (4) beinhaltet, um einen direkten Wärmeaustausch zwischen dem Faulbehälter (2) und dem Treibhaus (3) zu ermöglichen.

2. Integraler Faulbehälter und Treibhaus gemäß Anspruch 1, **dadurch gekennzeichnet, dass** der Faulbehältertank (4) einen Boden, eine von dem Boden aufragende nicht isolierte Seitenwand (5) und ein Dach (6) beinhaltet.

3. Integraler Faulbehälter und Treibhaus gemäß Anspruch 1 oder 2, **dadurch gekennzeichnet, dass** das Treibhaus (3) den Faulbehälter (2) umgibt.

4. Integraler Faulbehälter und Treibhaus gemäß Anspruch 3, **dadurch gekennzeichnet, dass** das Treibhaus (3) ein kreisförmiges Treibhaus (3) beinhaltet und der Faulbehälter (2) zentral in dem Treibhaus (3) gelegen ist.

5. Integraler Faulbehälter und Treibhaus gemäß einem der vorhergehenden Ansprüche, **dadurch gekennzeichnet, dass** sich der Faulbehälter (2) in dem Treibhaus (3) befindet, um einen Abschnitt höherer Temperatur (31) und einen Abschnitt niedrigerer Temperatur (32) in dem Treibhaus (3) zu definieren.

6. Integraler Faulbehälter und Treibhaus gemäß einem der vorhergehenden Ansprüche, **dadurch gekennzeichnet, dass** das Treibhaus (3) eine Glashaut (45) aus Kunststoff beinhaltet, der aus der Gruppe ausgewählt ist, die Polyethylen, glasfaserverstärktes Polyester und Polyvinylchlorid beinhaltet.

7. Integraler Faulbehälter und Treibhaus gemäß einem der vorhergehenden Ansprüche, **dadurch gekennzeichnet, dass** das Treibhaus (3) einen Versorgungsbereich (25) beinhaltet, von dem der integrale Faulbehälter und das Treibhaus (1) gewartet werden können, wobei sich ein Blockheizkraftwerk (22) in dem oder anliegend an den Versorgungsbereich (25) befindet.

8. Integraler Faulbehälter und Treibhaus gemäß Anspruch 7, **dadurch gekennzeichnet, dass** eine Trennvorrichtung (23) für den Faulbehälter (2) in dem Versorgungsbereich (25) untergebracht ist.

9. Ein Verfahren zum Ausfaulen von organischem Material und Heizen eines Treibhauses, wobei das Verfahren das Ausfaulen des organischen Materials in einem Faulbehälter (2) und das Anordnen des Faulbehälters (2), damit er sich in Wärmeaustauschzusammenspiel mit dem Treibhaus (3) befindet, beinhaltet, **dadurch gekennzeichnet, dass** das Verfahren das Anordnen eines anaeroben Faulbehälters (2), der einen nicht isolierten oder teilweise isolierten Faulbehältertank (4) beinhaltet, innerhalb des Treibhauses (3) beinhaltet, um den direkten Wärmeaustausch zwischen dem Faulbehälter (2) und dem Treibhaus (3) zu erleichtern.

# EP 1 100 867 B1

**Revendications**

1. Un digesteur et une serre solidaires comportant une serre (3) et un digesteur (2), **caractérisés en ce que** le digesteur (2) est un digesteur anaérobie (2) et est situé au sein de la serre (3), le digesteur anaérobie (2) comportant un réservoir de digesteur non isolé ou partiellement isolé (4) pour permettre un échange thermique direct entre le digesteur (2) et la serre (3).

2. Un digesteur et une serre solidaires tels que revendiqués dans la revendication 1 **caractérisés en ce que** le réservoir de digesteur (4) comporte une base, une paroi latérale non isolée (5) s'élevant depuis la base et un toit (6).

3. Un digesteur et une serre solidaires tels que revendiqués dans la revendication 1 ou la revendication 2 **caractérisés en ce que** la serre (3) entoure le digesteur (2).

4. Un digesteur et une serre solidaires tels que revendiqués dans la revendication 3 **caractérisés en ce que** la serre (3) comporte une serre circulaire (3), et le digesteur (2) est situé de façon centrale dans la serre (3).

5. Un digesteur et une serre solidaires tels que revendiqués dans n'importe quelle revendication précédente **caractérisés en ce que** le digesteur (2) est situé dans la serre (3) pour définir une portion de température plus élevée (31) et une portion de température plus basse (32) dans la serre (3).

6. Un digesteur et une serre solidaires tels que revendiqués dans n'importe quelle revendication précédente **caractérisés en ce que** la serre (3) comporte un revêtement vitrant (45) en plastique, lequel est sélectionné dans le groupe comportant le polyéthylène, le polyester chargé verre et le polychlorure de vinyle.

7. Un digesteur et une serre solidaires tels que revendiqués dans n'importe quelle revendication précédente **caractérisés en ce que** la serre (3) comporte une zone d'entretien (25) à partir de laquelle le digesteur et la serre solidaires (1) peuvent être entretenus, une unité de chauffage et d'énergie combinée (22) située dans ou adjacente à la zone d'entretien (25).

8. Un digesteur et une serre solidaires tels que revendiqués dans la revendication 7 **caractérisés en ce qu'**un séparateur (23) pour le digesteur (2) est logé dans la zone d'entretien (25).

9. Un procédé destiné à la digestion de matière organique et au chauffage d'une serre, le procédé comportant de digérer la matière organique dans un digesteur (2) et de situer le digesteur (2) de façon à ce qu'il soit en coopération d'échange thermique avec la serre (3), **caractérisé en ce que** le procédé comporte de placer un digesteur anaérobie (2) comportant un réservoir de digesteur non isolé ou partiellement isolé (4) au sein de la serre (3) pour faciliter l'échange thermique direct entre le digesteur (2) et la serre (3).

Fig. 1

Fig. 2

Fig. 3